(19) Europäisches Patentamt
European Patent Office
Office européen des brevets

(11) EP 1 765 295 B2

(12) **NEW EUROPEAN PATENT SPECIFICATION**
After opposition procedure

(45) Date of publication and mention of the opposition decision:
**09.09.2015 Bulletin 2015/37**

(45) Mention of the grant of the patent:
**12.09.2012 Bulletin 2012/37**

(21) Application number: **05758111.8**

(22) Date of filing: **30.06.2005**

(51) Int Cl.:
***A61K 9/00*** *(2006.01)* ***A61K 9/16*** *(2006.01)*
***A61K 9/50*** *(2006.01)* ***A61K 38/04*** *(2006.01)*

(86) International application number:
**PCT/JP2005/012517**

(87) International publication number:
**WO 2006/004167 (12.01.2006 Gazette 2006/02)**

(54) **A PROCESS FOR PRODUCING A SUSTAINED-RELEASE MICROCAPSULE**
EIN PROZESS ZUR HERSTELLUNG RETARDIERTER MIKROKAPSELN
UNE PROCÉDURE POUR PRODUIRE LES MICROCAPSULES À LIBÉRATION PROLONGÉE

(84) Designated Contracting States:
**AT BE BG CH CY CZ DE DK EE ES FI FR GB GR HU IE IS IT LI LT LU MC NL PL PT RO SE SI SK TR**

(30) Priority: **02.07.2004 JP 2004196831**
**14.09.2004 JP 2004267537**
**08.10.2004 JP 2004296635**
**31.01.2005 JP 2005023261**

(43) Date of publication of application:
**28.03.2007 Bulletin 2007/13**

(73) Proprietor: **Takeda Pharmaceutical Company Limited**
**Osaka-shi,**
**Osaka 541-0045 (JP)**

(72) Inventors:
- **FUTO, Tomomichi,**
**TAKEDA PHARMACEUTICAL CO., LTD.**
**Osaka-shi, Osaka 5328686 (JP)**
- **MUKAI, Kei,**
**TAKEDA PHARMACEUTICAL COMPANY LIMITED**
**Osaka-shi, Osaka 5328686 (JP)**
- **ARAI, Jiichi,**
**TAKEDA PHARMACEUTICAL COMPANY LTD.**
**Osaka-shi, Osaka 5328686 (JP)**

(74) Representative: **Kingsbury, Oliver William et al**
**Elkington and Fife LLP**
**Prospect House**
**8 Pembroke Road**
**Sevenoaks, Kent TN13 1XR (GB)**

(56) References cited:
**EP-A- 0 586 238**
**EP-A- 0 633 020**
**EP-A- 1 048 301**
**EP-A2- 1 142 567**
**WO-A-03/002091**

- **OGAWA Y ET AL: "CONTROLLED-RELEASE OF LEUPROLIDE ACETATE FROM POLYLACTIC ACID OR COPOLY(LACTIC/GLYCOLI) ACID MICROCAPSULES: INFLUENCE OF MOLECULAR WEIGHT AND COPOLYMER RATIO OF POLYMER" CHEMICAL AND PHARMACEUTICAL BULLETIN, PHARMACEUTICAL SOCIETY OF JAPAN, TOKYO, JP, vol. 36, no. 4, 1988, pages 1502-1507, XP001120337 ISSN: 0009-2363**

EP 1 765 295 B2

## Description

Technical Field

The present invention relates to a process for producing a sustained-release microcapsule comprising a physiologically active substance.

Background Art

For manufacturing microcapsules having improved spherical property, there have been a lot of literatures concerning the relationship between a method of preparing a secondary emulsion, that is to say, a W/O/W emulsion, and spherical property in microcapsule manufacturing technique using an in-water drying method. However, the effect of the temperature of an oil phase or an aqueous phase used in preparation of a primary emulsion, that is to say, a W/O emulsion, or the temperature of the primary emulsion on the spherical property has not been shown.

Microcapsules having poor spherical property are very difficult to administer due to their own non-uniform shape, which is a big problem in medical care. Specifically, in the case where a suspension for injection is produced using such microcapsules having poor spherical property, the needle penetrating property of the resulting suspension is small and results in poor aspiration of the suspension into an injection syringe in preparing for injection, and the like. In the case where the total amount of the suspension for injection cannot be aspirated into an injection syringe, the prescribed amount of a drug cannot be administered and as a result, the expected efficacy of the drug cannot be obtained.

Moreover, in the case where the suspension results in failure to pass through a needle when it is injected, the needle is clogged in the state of being stuck into the patient's body, which becomes an extremely big problem in medical care. Moreover, if a needle having a larger diameter is used for the purpose of obviating clogging of a needle, the pain of the patient is increased.

In order to avoid such a problem, a method for producing microcapsules comprising removal of microcapsules having undesired shapes by putting microcapsules through a sieve having a certain size is suggested, and however, it has a disadvantage of decreased yield.

JP-A 60-100516 discloses a sustained-release microcapsule of a water-soluble drug which is produced by an in-water drying method and which comprises a particle containing the water-soluble drug dispersed in a matrix having an average diameter of 2 to 200 $\mu$m consisting of a lactic acid-glycolic acid copolymer whose weight-average molecular weight is about 5,000 to 200,000 and in which lactic acid is present in about 100 to 50% by weight and glycolic acid is present in about 0 to 50% by weight.

JP-A 62-201816 discloses a method of producing a sustained-release microcapsule, which is characterized in that the viscosity of a W/O emulsion is adjusted to about 150 to 10,000 cp when a W/O/W emulsion is prepared.

JP-A 62-54760 discloses a coplymer or polymer of biodegradable polyoxycarboxylic acid ester in which the content of water-soluble oxycarboxylic acid is less than 0.01mol/100g as calculated on the assumption that it is monobasic acid and the weight-average molecular weight is about 2,000 to 50,000, and a sustained-release microcapsule for injection comprising the polymer.

JP-A 61-28521 discloses a lactic acid-glycolic acid copolymer consisting of about 50 to 95% by weight of lactic acid and about 50 to 5% by weight of glycolic acid, in which the weight-average molecular weight is about 5,000 to 30,000, a catalyst is not contained, and the dispersivity (as measured by a gel permeation chromatography method) is about 1.5 to 2, and a pharmaceutical containing the copolymer as a base.

JP-A 06-192068 discloses a method of producing sustained-release microcapsules characterized in that the microcapsules are heated at the glass-transition temperature of a polymer or above, at which temperature the particles of microcapsules are not attached to each other.

JP-A 04-218528 discloses a method of purifying biodegradable aliphatic polyester which comprises dissolving biodegradable aliphatic polyester containing a low-molecular-weight polymer having a molecular weight of 1,000 or less in an organic solvent, adding water to the solution to precipitate a high-molecular substance and then removing low molecular polymers having a molecular weight of 1,000 or less, and it discloses that 50 to 150 parts by volume of water is added to 100 parts by volume of an organic solvent.

WO 03/002091 discloses a sustained-release composition containing a lactic acid-glycolic acid copolymer having a ratio of weight-average molecular weight to number-average molecular weight of about 1.90 or less or a salt thereof, and a physiologically active substance, and a microsphere containing a lactic acid-glycolic acid copolymer having weight-average molecular weight of about 11,600 to about 14,000 or a salt thereof, and a LH-RH derivative or a salt thereof, and not containing gelatin.

In AAPS Pharmsci 1999, 1 (3) article 7, and European Journal of Pharmaceutics and Biopharmaceutics 50 (2000) 263-270, a microsphere containing leuprorelin acetate and a lactic acid-glycolic acid copolymer (50:50) having weight average molecular weight of 8.6 kDa is disclosed.

Disclosure of Invention

The present invention provides a process for producing a sustained-release microcapsule which has improved spherical property and/or needle penetrating property, which contains a high content of a physiologically active substance and does not contain a drug retaining substance such as gelatin, and which can maintain a stable releasing rate over a period of about one month by suppressing its initial excessive release.

The present inventors, under the above-described circumstances, diligently studied methods of producing a sustained-release microcapsule having great spherical property and/or needle penetrating property. As a result, they found the preferable temperature range of an oil phase used for preparation of a primary emulsion, that is, a W/O emulsion, and the preferable temperature range of the primary emulsion, and furthermore fond that a microcapsule which has great spherical property and/or needle penetrating property, which contains a high content of a physiologically active substance, and which can maintain a stable releasing rate over a period of about one month by suppressing its initial excessive release can be produced by using the preferable temperature ranges. Then, the present inventors further studied on the basis of these findings, and as a result, completed the present invention.

That is, the present invention provides:

[1] A process for producing a sustained-release microcapsule which comprises (i) a physiologically active substance and (ii) a lactic acid-glycolic acid copolymer in which the weight-average molecular weight (Mw) is about 8,000 to about 11,500, the ratio of the weight-average molecular weight (Mw) to the number-average molecular weight (Mn) is 2.3 to 3.1, and the compositional molar ratio of lactic acid to glycolic acid is 99.9/0.1 to 60/40, or a salt thereof, and which does not contain a drug retaining substance, and wherein the physiologically active substance is an LH-RH derivative; which comprises mixing (i) a solution containing a physiologically active substance and not containing a drug retaining substance and (ii) a solution adjusted to about 25 to about 35°C and containing a lactic acid-glycolic acid copolymer in which the weight-average molecular weight (Mw) is about 8,000 to about 11,500, the ratio of the weight-average molecular weight (Mw) to the number-average molecular weight (Mn) is 2.3 to 3.1, and the compositional molar ratio of lactic acid to glycolic acid is 99.9/0.1 to 60/40, or a salt thereof to prepare a W/O emulsion of about 25 to about 35°C; cooling the W/O emulsion to about 15 to about 20°C; dispersing the W/O emulsion into an aqueous phase to prepare a W/O/W emulsion; and then subjecting the W/O/W emulsion to in-water drying.

The above-described weight-average molecular weight (Mw) and number-average molecular weight (Mn) can be measured, for example, by the GPC method (1) described below in Reference Example 1.

The abbreviations of amino acid and protecting group and the other abbreviations as used herein are based on abbreviations in accordance with IUPAC-IUB (Commission on Biochemical Nomenclature) or conventional abbreviations in the art. If amino acids may have the optical isomers, they are represented in the L-configuration unless otherwise specified.

Furthermore, the abbreviations used herein represent the following meanings:

DSer(tBu): O-tert-butyl-D-serine residue
D2Nal: D-3-(2-naphtyl)alanine residue
DHis(imBzl): Nim-benzyl-D-histidine residue
NacD2Nal: N-acetyl-D-3-(2-naphtyl)alanyl
D4ClPhe: D-3-(4-chlorophenyl)alanyl
D3Pal: D-3-(3-pyridyl)alanyl
NmeTyr: N-methyltyrosyl
DLys(Nisp): D-(epsilon -N-nicotinoyl)lysyl
Lys(Nisp): (epsilon-N-isopropyl)lysyl
DhArg(Et2): D-(N,N'-diethyl)homoarginyl

Mode for Carrying Out the Invention

A physiologically active substance used in the present invention is an LHRH derivative, including an LHRH (luteinizing hormone-releasing hormone) agonist or an LHRH antagonist.

An LHRH agonist includes, for example, a peptide represented by the formula:

(Pyr) Glu-$R^1$-Trp-Ser-$R^2$-$R^3$-$R^4$-Arg-Pro-$R^5$ (I)

[wherein, $R^1$ represents His, Tyr, Trp or p-$NH_2$-Phe; $R^2$ represents Tyr or Phe; $R^3$ represents Gly or a D-type amino acid residue which may be substituted; $R^4$ represents Leu, Ile or Nle; and $R^6$ represents Gly-NH-$R^6$ (wherein $R^6$ represents

hydrogen or alkyl having or not having hydroxyl) or NH-$R^7$ (wherein $R^7$ represents hydrogen, alkyl having or not having amino or hydroxyl, or ureide (-NH-CO-$NH_2$))] or a salt thereof.

In the above formula (I), the D-type amino acid residue represented by $R^3$ includes, for example, α-D-amino acid containing up to 9 carbon atoms (for example, D-Leu, Ile, Nle, Val, Nval, Abu, Phe, Phg, Ser, Thr, Met, Ala, Trp, α-Aibu and the like) and the like. A substituent for $R^3$ includes, for example, tert-butyl, tert-butoxy, tert-butoxycarbonyl, methyl, dimethyl, trimethyl, 2-naphtyl, indol-3-yl, 2-methylindolyl, benzyl-imidazol-2-yl and the like.

In the formula (I), alkyl represented by $R^6$ or R is preferably, for example, $C_{1-4}$ alkyl, and the examples include methyl, ethyl, prophyl, isopropyl, butyl, isobutyl, sec-butyl, and tert-butyl.

A salt of a peptide represented by the formula (I) [hereinafter, it may be abbreviated as peptide (I)] includes, for example, salts of acid (for example, carbonate, bicarbonate, acetate, trifluoroacetate, propionate, succinate and the like) and metal complex compounds (for example, cupper complex, zinc complex and the like).

As the peptide represented by the formula (I) or a salt thereof, a peptide represented by the formula:

5-oxo-Pro-His-Trp-Ser-Tyr-Y-Leu-Arg-Pro-Z

[wherein Y represents DLeu, DAla, DTrp, DSer(tBu), D2Nal o: DHis (ImBzl), and Z represents NH-$C_2H_5$ or Gly-$NH_2$] or a salt thereof is preferred, and furthermore a peptide represented by the formula:

5-oxo-Pro-His-Trp-Ser-Tyr-DLeu-Leu-Arg-Pro-NH-$C_2H_5$

or the acetate thereof is preferred.

The peptide (I). or its salt can be produced by, for example, a method described in USP No. 3,853,837, USP No. 4,008,209, USP No. 3,972,859, British Patent No. 1,423,083 Proceedings of the National Academy of Sciences of the United States of America, Vol.78, pp.6509-6612 (1981) or the like, or a similar method to that.

The peptide (I) is preferably any one of peptides represented by the following formulas (a)-(j) or the like, or a salt thereof.

(a) Leuprorelin [a peptide represented by the formula (I) wherein $R^1$ = His, $R^2$ = Tyr, $R^3$ = D-Leu, $R^4$ = Leu, $R^5$ = $NHCH_2$-$CH_3$];
(b) Gonadrelin

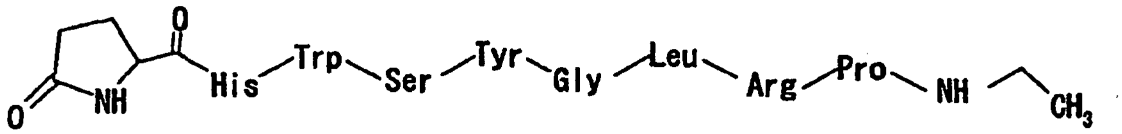

[German Patent No. 2213737];
(c) Buserelin

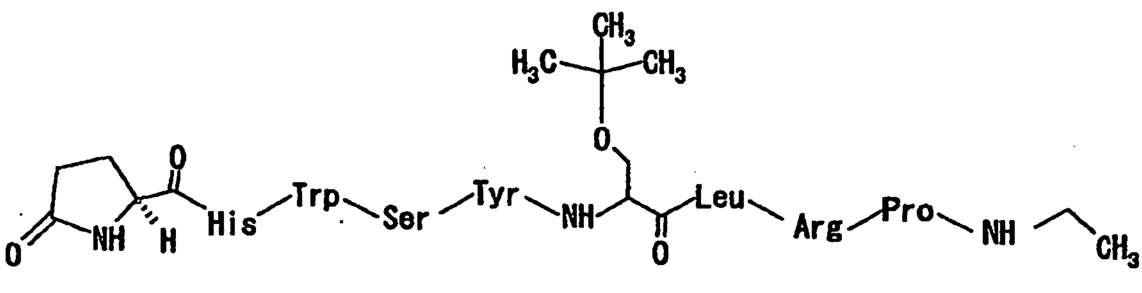

[USP No. 4,024,248, German Patent No. 2,438,352, JP-A 51-41359];
(d) Triptorelin

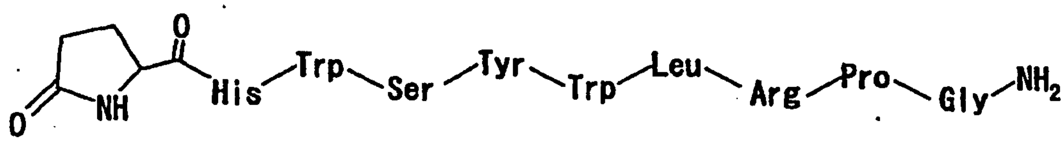

[USP No. 4,010,125, JP-A 52-31073];
(e) Goserelin

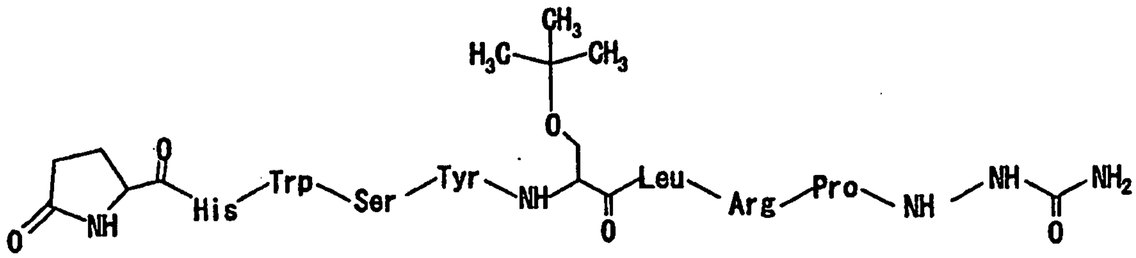

[USP No. 4,100,274, JP-A 52-136172];
(f) Nafarelin

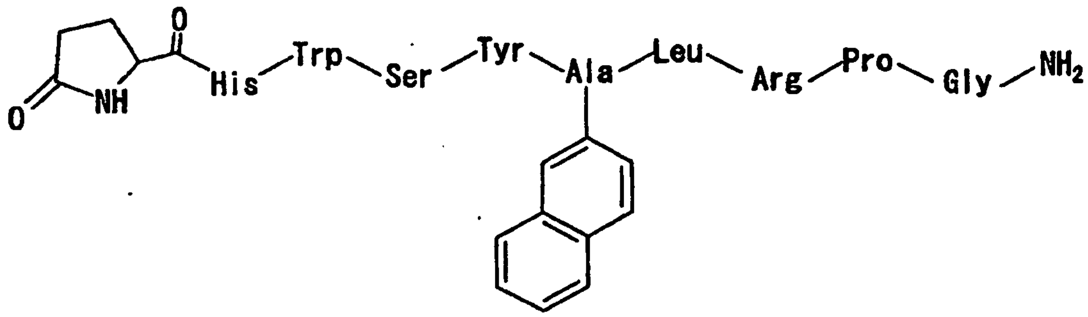

[USP No. 4,234,571, JP-A 55-164663, JP-A 63-264498, JP-A 64-25794];
(g) Histrelin

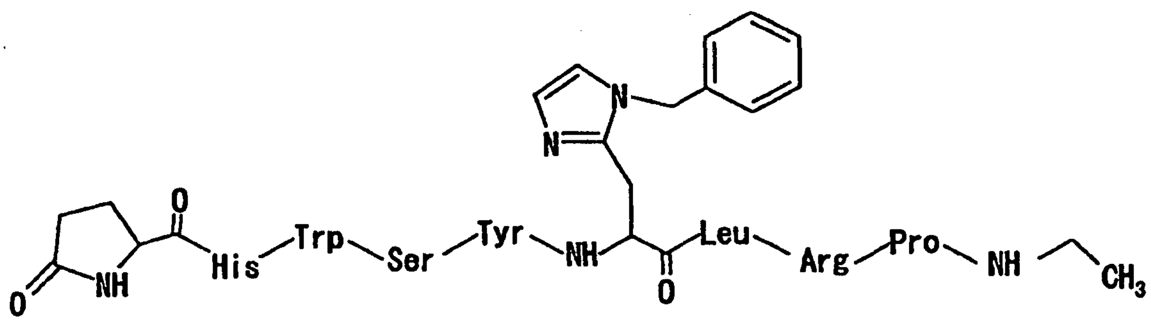

(h) Deslorelin

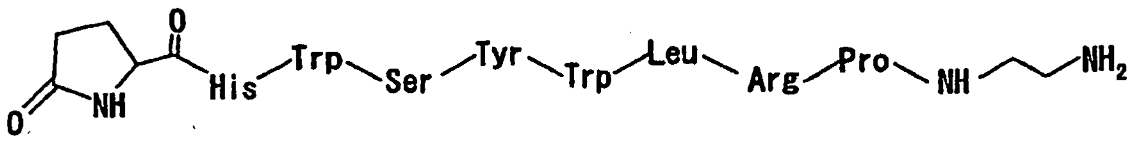

[USP No. 4,569,967, USP No. 4,248,439];
(i) Meterelin

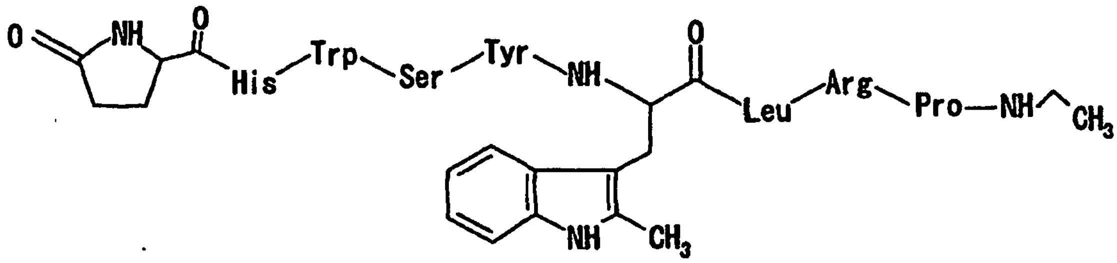

[WO9118016];
(j) Lecirelin

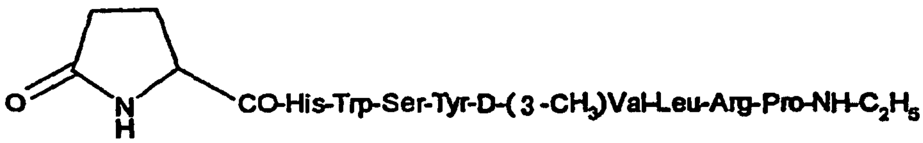

[Belgian Patent No. 897455, JP-A 59-59654].

In the above formulas **(c)-(j),** the amino acid corresponding to $R^3$ in the formula (I) is D-form.

The peptide (I) or its salt is more preferably leuprorelin or leuprorelin acetate. Herein, leuprorelin acetate is acetate salt of leuprorelin.

An LHRH antagonist includes, for example, those disclosed in USP No. 4,086,219, USP No. 4,124,577, USP No. 4,253,997 and USP No. 4,317,815, and a peptide represented by the formula:

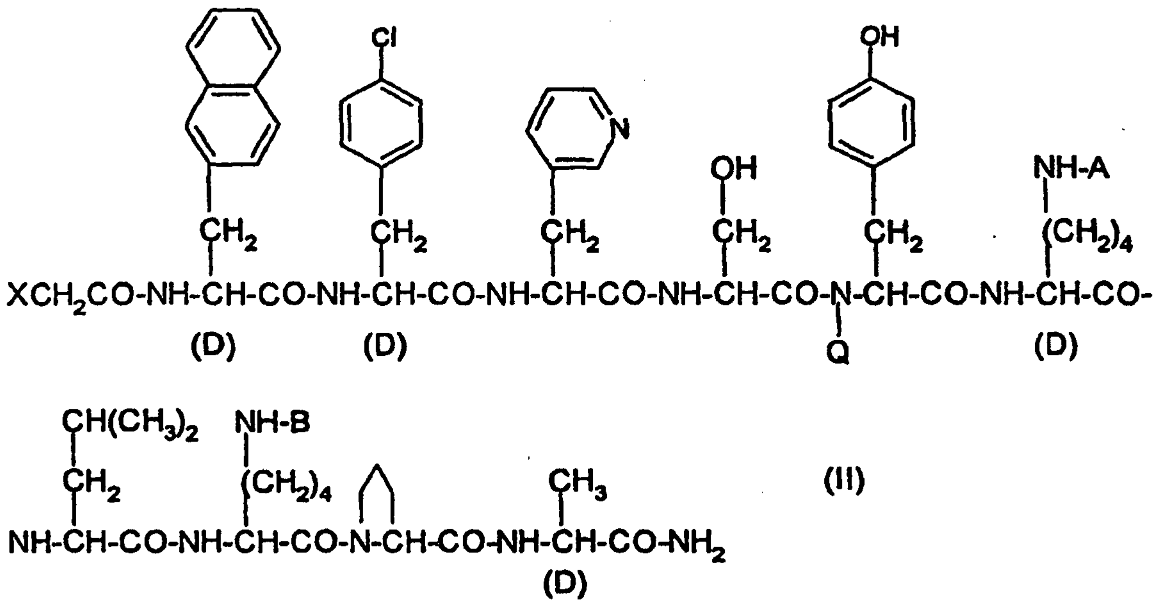

(II)

wherein, X represents hydrogen or tetrahydrofurylcarboxamide, Q represents hydrogen or methyl, A represents nicotinoyl or N,N'-diethylamidino, and B represents isopropyl or N,N'-diethylamidino, [hereinafter, it may be abbreviated as peptide (II)] or its salt.

In the formula (II), X is preferably tetrahydrofurylcarboxamide, more preferably (2S)-tetrahydrofurylcarboxamide. Moreover, A is preferably nicotinoyl. B is preferably isopropyl.

In the case where the peptide (II) has one or more species of asymmetric carbon atoms, two or more kinds of optical isomers exist. The peptide (II) may be used as such optical isomers or as mixture of such optical isomers.

A salt of the peptide (II) may be preferably a pharmacologically acceptable salt. Such a salt includes salts with inorganic acid (for example, hydrochloride, sulfate, nitrate and the like), salts with organic acid (for example, carbonate, bicarbonate, succinate, acetate, propionate, trifluoroacetate and the like) and the like. A salt of the peptide (II) is more preferably a salt with organic acid (for example, carbonate, bicarbonate, succinate, acetate, propionate, trifluoroacetate and the like). A salt of the peptide (II) is still more preferably a salt with acetic acid. These salts may be mono- to tri-salts.

The peptide (II) or its salt is preferably represented by any one of the following formulas (1)-(4):

(1)

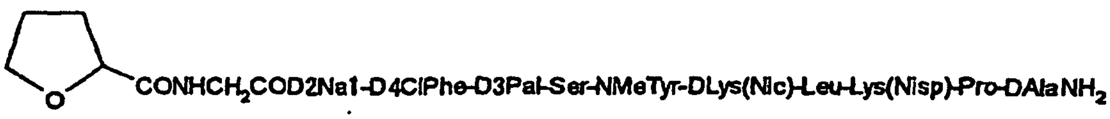

(hereinafter, the S-isomer of said peptide is abbreviated as compound A);

(2)

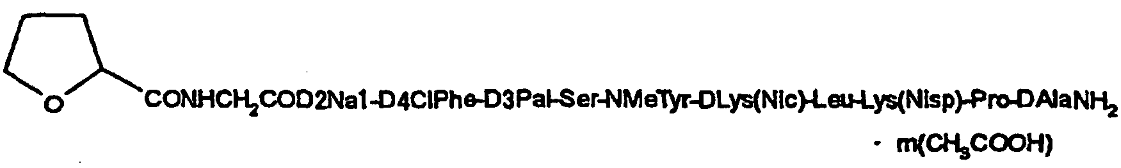

[wherein, m represents a real number of 1 to 3];

(3) NcD2Nal-D4ClPhe-D3Pal-Ser-Tyr-DhArg($Et_2$)-Leu-hArg($Et_2$)-Pro-DAlaNH$_2$;

(4) NcD2Nal-D4ClPhe-D3Pal-Ser-Tyr-DhArg($Et_2$) -Leu-hArg($Et_2$) - Pro-DAlaNH$_2$·n($CH_3COOH$)

[wherein, n represents a real number of 1 to 3].

The above formulas (2) and (4) represent a salt or solvate.

The peptide (II) or its salt is more preferably represented by the above formula (1) or (2), and in particular, it is preferably the S-isomer. Hereinafter, the S-isomer of a peptide represented by the above formula (1) is abbreviated as compound A1.

The peptide (II) or its salt can be produced by a per se known method, for example, a method described in JP-A 3-101695 (EP-A 413209), Journal of Medicinal Chemistry, vol.35, pp.3942 (1992) or the like, or a similar method to that.

The physiologically active substance may be used as it is or in the form of a pharmacologically acceptable salt. For example, in the case where the physiologically active substance has a basic group such as amino, it may be used in the form of a salt with an inorganic acid (for example, hydrochloric acid, sulfuric acid, nitric acid or the like) or an organic acid (for example, carbonic acid, succinic acid, or the like). In the case where the physiologically active substance has an acidic group such as carboxy, it may be used in the form of a salt with an inorganic base (for example, an alkali metal such as sodium, potassium or the like) or an organic base (for example, an organic amine such as triethylamine or the like, a basic amino acid such as arginine or the like).

A biodegradable polymer used in the present invention is a lactic acid-glycolic acid copolymer in which the weight-average molecular weight (Mw) is about 8,000 to about 11,500, the ratio of the weight-average molecular weight (Mw) to the number-average molecular weight (Mn) is 2.3 to 3.1, and the compositional molar ratio of lactic acid to glycolic acid is 99.9/0.1 to 60/40, or a salt thereof.

A salt of the lactic acid-glycolic acid copolymer includes, for example, a salt with an inorganic base (for example, an alkali metal such as sodium or potassium, an alkaline earth metal such as calcium or magnesium, or the like), a salt with an organic base (for example, an organic amine such as triethylamine, a basic amino acid such as arginine, or the like), a salt with a transition metal (for example, zinc, iron, cupper or the like), a complex salt and the like.

The ratio (Mw/Mn) of the weight-average molecular weight (Mw) of the lactic acid-glycolic acid copolymer to the number-average molecular weight (Mn) of the lactic acid-glycolic acid copolymer is 2.3 to 3.1.

The compositional ratio (mol%) of the lactic acid-glycolic acid copolymer is preferably 99/1 to 60/40, more preferably 90/10 to 60/40, and still more preferably 80/20 to 60/40, particularly preferably 80/20 to 70/30, and most preferably 75/25.

The weight average molecular weight of the above-described lactic acid-glycolic acid copolymer is usually about 8,000 to about 11,500, preferably about 9,000 to about 11,500, and more preferably about 9,500 to 11,000.

A weight-average molecular weight and a number-average molecular weight, as used herein, refer to the molecular weights (weight-average molecular weight and number-average molecular weight) that is measured by gel permeation chromatography (GPC) using several kinds of polystyrenes having certain weight-average molecular weights as standard substances, followed by calibration with polystyrene. Dispersivity as used herein refers to the dispersivity calculated from the molecular weights obtained as described above. A column and a mobile phase used in the measurement can be appropriately selected. Alternatively, a lactic acid-glycolic acid copolymer is dissolved in dichloromethane, added water, and partitioned. The dichloromethane layer is titrated with an ethanolic potassium hydroxide solution using an automatic titrating apparatus and the amount of terminal carboxylic acid is calculated, whereby the number-average molecular weight can be calculated. Hereinafter, this is expressed as a number-average molecular weight by means of terminal group quantitation. A number-average molecular weight by means of terminal group quantitation is absolute value, while a number-average molecular weight by means of GPC measurement is relative value varying depending on assay and analysis conditions (for example, the kind of a mobile phase, the kind of a column, a standard substance, selection of the width of slice, selection of a baseline and the like). Therefore, it is difficult to digitize a number-average molecular weight univocally. However, for example, in the case of a polymer having a free carboxyl group at the terminal which is synthesized from lactic acid and glycolic acid by a dehydration polycondensation method without a catalyst, the number-average molecular weight by means of GPC measurement and the number-average molecular weight by means of terminal group quantitation are approximately the same value. As for the number-average molecular weight of this lactic acid-glycolic acid copolymer, "approximately the same" means that the number-average molecular weight by means of terminal group quantitation is in the range from about 0.2 to about 1.5 times, preferably from about 0.3 to about 1.2 times the number-average molecular weight by means of GPC measurement.

Herein, the GPC method (1) described in Reference Example 1 is a GPC method comprising using 8 polystyrene standard products whose weight-average molecular weights (Mw) evaluated by a GPC method are 98,900, 37,200, 17,100, 9, 490, 5,870, 2, 500, 1,051, and 495 respectively.

The GPC method (2) described in Reference Example 1 is a GPC method comprising using a total of 8 polystyrene standard products consisting of 6 polystyrene standard products whose weight-average molecular weights (Mw) evaluated by a light scattering method are 96,400, 37,900, 18,100, 10,200, 5, 970 and 2,630 respectively and 2 polystyrene standard products whose weight-average molecular weights (Mw) measured by a GPC method whose are 1,051 and 495 respectively.

The weight-average molecular weight and the number-average molecular weight of the above-described lactic acid-glycolic acid copolymer or a salt thereof used in the present invention can be measured by, for example, the GPC method (1) described in Reference Example 1.

More specifically, the following lactic acid-glycolic acid copolymers and the like are preferably used.

(1) Lactic acid-glycolic acid copolymer (lactic acid/glycolic acid = 75/25, Mw = about 10,300, Mn = about 4,000, Mw/Mn ratio = 2.6 (value by the GPC method (1) of Reference Example 1)).

(2) Lactic acid-glycolic acid copolymer (lactic acid/glycolic acid = 75/25, Mw = about 10,400, Mn = about 4,100, Mw/Mn ratio = 2.5 (value by the GPC method (1) of Reference Example 1)).

The degradation and/or elimination rate of a lactic acid-glycolic acid copolymer varies greatly depending on the composition or molecular weight of the polymer. Generally, the lower the percentage of glycolic acid in the polymer is, the more the degradation/elimination is delayed. Therefore, a releasing period of a drug can be extended by lowering the percentage of glycolic acid in the polymer or by increasing the molecular weight of the polymer. Conversely, a releasing period can be shortened by increasing the percentage of glycolic acid or by decreasing the molecular weight. In order to produce a long-period (for example, 1-12 months, preferably 1-6 months)-type sustained-release preparation, it is preferable to use a lactic acid-glycolic acid copolymer having a compositional ratio and a weight-average molecular weight in the above-mentioned range. When a lactic acid-glycolic acid copolymer which is degraded more rapidly than a lactic acid-glycolic acid copolymer having a compositional ratio and a weight-average molecular weight in the above-mentioned range is selected, it is difficult to suppress the initial burst of the resulting sustained-release preparation. In contrast, when a lactic acid-glycolic acid copolymer which is degraded more slowly than a lactic acid-glycolic acid copolymer having a compositional ratio and a weight-average molecular weight in the above-mentioned range is selected, the resulting preparation tends to have a period during which an effective amount of a drug is not released after administration.

A lactic acid-glycolic acid copolymer can be produced by, for example, non-catalytic dehydration polycondensation from lactic acid and glycolic acid (JP-A 61-28521) or ring opening polymerization using a catalyst from cyclic forms such as lactide and glycolide (Encyclopedic Handbook of Biomaterials and Bioengineering Part A: Materials, Volume 2, Marcel Dekker, Inc. 1995).

Although a polymer synthesized by ring opening polymerization is a polymer having no carboxyl group, such a polymer may be subjected to chemical treatment to change the terminal into free carboxyl, which may be also used (J. Controlled Release, Vol.41, pp.249-257, 1996).

The above-described lactic acid-glycolic acid copolymer having free carboxyl at the terminal can be easily produced by a known method (for example, non-catalytic dehydration polycondensation method, see JP-A 61-28521). Further, a polymer having free carboxyl at any position that is not limited to the terminal can be produced by a known method (for example, see WO94/15587).

The lactic acid-glycolic acid copolymer whose terminal is changed into free carboxyl by chemical treatment after ring opening polymerization may be commercially available, for example, from Boehringer Ingelheim KG.

Furthermore, the lactic acid-glycolic acid copolymer produced by ring opening polymerization is hydrolyzed in the presence of an acid or a base in accordance with a known method. In addition, the hydrolysis is performed in the presence of water.

Herein, the acid includes inorganic acids such as hydrochloric acid, nitric acid, sulfuric acid and phosphoric acid, and organic acids such as lactic acid, acetic acid, tartaric acid, citric acid and succinic acid. The base includes alkali metal hydroxide such as sodium hydroxide and potassium hydroxide, and alkali metal carbonate such as sodium carbonate and potassium carbonate. In the case where the hydrolysis is performed in the presence of a base, release of a physiologically active substance from the sustained-release microcapsule is affected by the remaining amount of the base. Therefore, it is preferable that the hydrolysis is performed in the presence of an acid.

The hydrolysis is usually performed in a solvent which have no adverse effect on the reaction. Such a solvent includes alcohols such as methanol, ethanol and propanol, ethers such as tetrahydrofuran, dioxane, diethyl ether and diisopropyl ether, water, and a mixed solvent thereof. Alternatively, an excessive amount of the above-described acid or base may be used as a solvent.

A temperature upon the hydrolysis is, for example, about 0 to about 100°C, preferably about 10 to about 100°C.

Since the time necessary for the hydrolysis varies depending on the weight-average molecular weight of a lactic acid-glycolic acid copolymer produced by ring opening polymerization, the kind of an acid or a base, the kind of a solvent, temperature and the like, it may be appropriately determined by collecting a portion of a lactic acid-glycolic acid copolymer during hydrolysis and then measuring the weight-average molecular weight of the collected lactic acid-glycolic acid copolymer. The time necessary for the hydrolysis is not particularly limited, but it is, for example, about 1 hour to about 10 days, preferably about 10 hours to about 5 days.

From a lactic acid-glycolic acid copolymer produced by ring opening polymerization, only a sustained-release

microcapsule that induces a great initial burst can be produced. However, from a hydrolyzed lactic acid-glycolic acid copolymer, that is to say, the lactic acid-glycolic acid copolymer used in the present invention, a sustained-release microcapsule that induces a small initial burst can be produced.

Preferably, the hydrolyzed lactic acid-glycolic acid copolymer is further subjected to a purification step. The purification step is performed by dissolving the hydrolyzed lactic acid-glycolic acid copolymer in an organic solvent, pouring the obtained solution into water or a mixed solution of water and a water-soluble organic solvent and then separating a precipitated lactic acid-glycolic acid copolymer.

The organic solvent includes, for example, halogenated hydrocarbons (for example, dichloromethane, chloroform, chloroethane, dichloroethane, trichloroethane, carbon tetrachloride and the like), ketones (for example, acetone and the like), ethers (for example, tetrahydrofuran, ethyl ether, isopropyl ether and the like), esters (for example, ethyl acetate, butyl acetate and the like), aromatic hydrocarbons (for example, benzene, toluene, xylene and the like) and the like. The amount of the organic solvent used is, for example, about 3 to about 20 times (w/v) the amount of the hydrolyzed lactic acid-glycolic acid copolymer used.

The water-soluble organic solvent includes, for example, acetone, methanol, ethanol, tetrahydrofuran, acetonitrile and the like. The amount of water or a mixed solution of water and the water-soluble organic solvent to be used is not particularly limited, but it is usually a greatly excessive amount relative to the amount of hydrolyzed lactic acid-glycolic acid copolymer used.

The temperature in the purification step is usually about 0 to about 90°C, preferably about 20 to about 70°C.

Water-soluble low-molecular compounds (for example, compounds having a weight-average molecular weight of about 1,000 or less) can be removed by carrying out the above-described purification step. When the lactic acid-glycolic acid copolymer obtained via such purification step is used to produce a sustained-release microcapsule, the incorporation rate (trapping rate) of a physiologically active substance into the resulting sustained-release microcapsule can be enhanced, and moreover, the resulting sustained release preparation can induce a reduced initial burst.

Furthermore, a lactic acid-glycolic acid copolymer that does not substantially contain a harmful catalyst used in ring opening polymerization (for example, a zinc compound such as zinc oxide or a tin compound such as stannous octanoate) can be produced by subjecting a lactic acid-glycolic acid copolymer produced by ring opening polymerization to hydrolysis and the purification step.

A drug retaining substance is a substance characterized in that it is water-soluble, hardly soluble in an organic solvent if a oil phase, becomes a semi-solid that is already highly viscous in the state of being dissolved in water, or the viscosity is remarkably increased by some extrinsic factors such as temperature, pH, a metal ion (for example, $Cu^{2+}$, $Al^{3+}$, $Zn^{2+}$ and the like), organic acid (for example, tartaric acid, citric acid, tannic acid and the like) or its salt, a chemical condensing agent (for example, glutaraldehyde, acetoaldehyde and the like) to become a semi-solid or solid matrix.

Examples of a drug retaining substance are natural and synthetic gums and high-molecular compounds.

Natural gums include acacia gum, gum arabic, Irish moss, karaya gum, tragacanth gum, guaiac gum, xanthan gum, and locust bean gum. Natural high-molecular compounds include protein such as casein, gelatin, collagen, albumin (for example, human serum albumin), globulin, and fibrin, and carbohydrate such as cellulose, dextrin, pectin, starch, agar, and mannan. These may be as they are, or may be partially chemically modified synthetic gums, for example, the above-mentioned natural gums which have been esterified or etherized (for example, methylcellulose, ethylcellulose, carboxylmethylcellulose, gelatin succinate and the like), or hydrolyzed (for example, sodium alginate, sodium pectinate and the like), or their salts.

Synthetic high-molecular compounds include, for example, polyvinyl compounds (for example, polyvinylpyrrolidone, polyvinyl alcohol, polyvinyl methyl ether, polyvinyl ether and the like), polycarboxylic acid (for example, polyacrylic acid, polymethacrylic acid, Carbopol (Goodrich, Co., Ltd.) and the like), polyethylene compounds (for example, polyethylene glycol and the like), polysaccharide (for example, polysucrose, polyglucose, polylactose and the like), and their salts.

In addition, such substances that can proceed in condensation and crosslinking by the above-mentioned extrinsic factors to result in high-molecular compounds is also included.

Among these compounds, inter alia, gelatin, albumin, pectin or agar, in particular, gelatin is suitable for a drug retaining substance.

The sustained-release composition is in the form of a sustained-release microcapsule.

Confirmation of the form can be performed, for example, by observation with a scanning electron microscope.

The sustained-release microcapsule may contain fine particles (that is, microspheres) containing a physiologyically active substance and a lactic acid-glycolic acid copolymer or a salt thereof. Specific examples of the fine particle include a microcapsule containing one core of a physiologically active substance in one particle, a polynuclear microcapsule containing a plurality of cores of a physiologically active substance in one particle, and a microparticle in which a molecular physiologically active substance as a solid solution is dissolved or dispersed in a raw lactic acid-glycolic acid copolymer or a salt thereof.

The content of a physiologically active substance in the sustained-release microcapsule varies depending on the kind of the physiologically active substance, the desired pharmacological effect, duration of the effect and the like,

and for example, it is about 0.01 to about 50%(w/w), preferably about 0.1 to about 30%(w/w), more preferably about 5 to about 24% (w/w).

Hereinafter, a process for producing a sustained-release microcapsule which is a representative example of the present invention will be described in detail.

The sustained-release microcapsule is produced by mixing (i) a solution containing a physiologically active substance and not containing a drug retaining substance and (ii) a solution adjusted to about 25 to about 35°C and containing a lactic acid-glycolic acid copolymer or a salt thereof (hereinafter, abbreviated as the biodegradable polymer) in which the weight-average molecular weight (Mw) is about 8,000 to about 11,500, the ratio of the weight-average molecular weight (Mw) to the number-average molecular weight (Mn) is 2.3 to 3.1, and the compositional molar ratio of lactic acid to glycolic acid is 99.9/0.1 to 60/40, to prepare a W/O emulsion of about 25 to about 35°C (primary emulsification); cooling the W/O emulsion to about 15 to about 20°C; dispersing the W/O emulsion into an aqueous phase to prepare a W/O/W emulsion (secondary emulsification); and then subjecting the W/O/W emulsion to in-water drying.

The W/O emulsion that comprises a solution containing a physiologically active substance and not containing a drug retaining substance as an inner aqueous phase, and a solution adjusted to about 25 to about 35°C and containing the biodegradable polymer as an oil phase can be produced as follows.

First, a physiologically active substance is dissolved in water (preferably, distilled water for injection) at a concentration of about 0.001 to about 90% (w/w), preferably about 0.01 to about 80% (w/w), more preferably about 1 to about 70% (w/w), particularly preferably about 50%, to form an inner aqueous phase.

To the inner aqueous phase may be added a pH adjusting agent such as carbonic acid, acetic acid, oxalic acid, citric acid, phosphoric acid, hydrochloric acid, sodium hydroxide, arginine, lysine, or a salt thereof, for the purpose of maintaining the stability and solubility of the physiologically active substance. In addition, may be added a stabilizing agent for the physiologically active substance, such as albumin, gelatin, trehalose, citric acid, sodium ethylenediamine tetraacetate, dextrin, cyclodextrin ($\alpha$-, $\beta$-, $\gamma$-) and a derivative thereof (for example, maltosyl $\beta$-cyclodextrin, $\beta$-cyclodextrin sulfobutyl ether or the like), sodium hydrogen sulfite, a polyol compound such as polyethylene glycol, a surfactant such as polyoxyethylene sorbitan fatty acid ester [for example, Tween 80, Tween 60 (Kao Corporation, Japan)] or a polyoxyethylene caster oil derivative [for example, HCO-60, HCO-70 (Nikko Chemicals Co., Ltd., Japan)], parahydroxybenzoic acid ester (for example, methylparaben, propylparaben or the like), benzyl alcohol, chlorobutanol, thimerosal or the like.

The inner aqueous phase thus obtained is mixed with a solution adjusted to about 25 to about 35°C and containing the biodegradable polymer (oil phase). The mixture is subjected to an emulsification step to prepare a W/O emulsion.

The solution containing the biodegradable polymer (oil phase) to be used is a solution of the biodegradable polymer in an organic solvent. The organic solvent may be a solvent which has a boiling point of about 120°C or lower, is hydrophobic and can dissolve the biodegradable polymer, and includes, for example, halogenated hydrocarbons (for example, dichloromethane (methylene chloride), chloroform, chloroethane, dichloroethane, trichloroethane, carbon tetrachloride and the like), fatty acid esters (for example, ethyl acetate, butyl acetate and the like), ethers (for example, ethyl ether, isopropyl ether and the like), aromatic hydrocarbons (for example, benzene, toluene, xylene and the like), and the like. Two or more kinds of these organic solvents may be mixed at an appropriate ratio and used. The organic solvent is preferably methylene chloride.

The concentration of the biodegradable polymer in the organic solvent varies depending on the kind and molecular weight of the biodegradable polymer, and the kind of the organic solvent, and it is usually about 0.01 to about 90% (w/w), preferably about 0.1 to about 80% (w/w), more preferably about 1 to 70% (w/w), particularly preferably about 35%.

In order to change compatibility with the inner aqueous phase, distribution of the organic solvent into the outer aqueous phase, and volatilization of the organic solvent, a hydrophilic organic solvent such as ethanol, acetonitrile, acetone or tetrahydrofuran may be partially added to the oil phase. Moreover, in order to dissolve or stabilize the interior physiologically active substance, a surfactant such as sucrose fatty acid ester may be added.

The oil phase thus obtained is usually used after removing bacteria and dusts by filtration using a filter. The solution containing the biodegradable polymer may be stored in a sealed container at room temperature or in cold places, depending on the stability of the biodegradable polymer.

The mixing ratio between the solution containing a physiologically active substance and not containing a drug retaining substance and the biodegradable polymer solution is about 0.1 to about 1000 parts by weight, preferably about 1 to about 100 parts by weight, more preferably about 1 to about 20 parts by weight, particularly preferably about 10 parts by weight of the latter per 1 part by weight of the former.

The mixing ratio of a physiologically active substance to the biodegradable polymer is about 0.01 to about 50% (w/w), preferably about 0.5 to about 40% (w/w), more preferably about 0.1 to about 30% (w/w), particularly preferably about 10%, depending on the kind of the physiologically active substance, the desired pharmacological effect, duration period of the effect and the like.

The emulsification step is carried out by a known dispersing method, for example, an intermittent vibration

method, a method using a stirrer such as propeller-type stirrer or a turbine-type stirrer, a colloid mill method, a homogenizer method, an ultrasound irradiation method or the like.

Then, the solution containing a physiologically active substance and not containing a drug retaining substance and the solution containing the biodegradable polymer are mixed at about 25 to about 35°C, preferably about 27 to about 33°C. By this temperature adjustment, a sustained-release microcapsule having improved spherical property and/or improved needle penetrating property can be produced.

A preferable aspect of the emulsification step will be described. For example, first, the solution containing the biodegradable polymer is added to a container containing the solution containing a physiologically active substance and not containing a drug retaining substance. Then, the container is vibrated or swung, thereby rough emulsification is performed. In rough emulsification, it is preferable that the temperature of a mixture of the solution containing a physiologically active substance and not containing a drug retaining substance and the solution containing the biodegradable polymer is adjusted to about 25 to about 35°C, preferably about 27 to about 33°C.

Generally, a purpose of rough emulsification is to facilitate the next emulsification step (precise emulsification), and the stirring time, and the vibration and swinging number are not particularly defined. Therefore, if precise emulsification can be carried out uniformly, the rough emulsification step may be omitted.

Next, the mixture after the rough emulsification is subjected to an emulsification step (precise emulsification) using a propeller-type stirrer or the like. In precise emulsification, it is preferable that the temperature of a mixture of the solution containing a physiologically active substance and not containing a drug retaining substance and the solution containing the biodegradable polymer is adjusted to about 25 to about 35°C, preferably about 27 to about 33°C. By this temperature adjustment, a sustained-release microcapsule having improved spherical property and/or improved needle penetrating property can be produced. The emulsification time of the precise emulsification step can be selected depending on the properties of the physiologically active substance and the biodegradable polymer, and generally, it is about 0.1 to about 60 minutes.

The volume of the oil phase to be mixed is about 1 to about 1000 times, preferably about 2 to about 100 times, more preferably about 3 to 10 times the volume of the inner aqueous phase.

The viscosity of the resulting W/O emulsion is generally in the range of about 10 to about 10,000 cp, preferably about 100 to 5,000 cp, particularly preferably about 500 to about 2,000 cp at about 12 to 25°C.

It is preferable that the W/O emulsion obtained by precise emulsification is cooled in a water bath or the like at 0 to 18°C to adjust the temperature of the W/O emulsion to about 0 to about 30°C, preferably about 10 to about 25°C, more preferably about 15 to 20°C.

Subsequently, the W/O emulsion thus obtained is dispersed in an aqueous phase (hereinafter, abbreviated as outer aqueous phase) to prepare a W/O/W emulsion. The W/O/W emulsion is subjected to in-water drying to produce a sustained-release microcapsule.

An emulsifier may be added to the above-described outer aqueous phase. The emulsifier may be any emulsifier that usually forms a stable W/O emulsion, and includes, for example, anion surfactants (for example, sodium oleate, sodium stearate, sodium lauryl sulfate and the like), nonionic surfactants (for example, Tween 80, Tween 60, HCO-60, HCO-70 and the like), polyvinyl alcohol, polyvinylpyrrolidone, gelatin and the like. Two or more kinds of these emulsifiers may be mixed at an appropriate ratio and used. In the process of the present invention, polyvinyl alcohol is preferably used as an emulsifier.

The concentration of an emulsifier in the outer aqueous phase is, for example, about 0.001 to about 20%, preferably about 0.01 to about 10%, more preferably about 0.05 to about 5%, particularly preferably about 0.1%.

An osmotic pressure regulating agent may be added to the above-described outer aqueous phase. The osmotic pressure regulating agent may be any agent that exhibits an osmotic pressure when it is put into an aqueous solution.

The osmotic pressure regulating agent includes, for example, polyhydric alcohols, monohydric alcohols, monosaccharides, disaccharides, oligosaccharides and amino acids and their derivatives, and sodium chloride.

The polyhydric alcohols include, for example, trihydric alcohols such as glycerin, pentahydric alcohols such as arabitol, xylitol and adonitol, and hexahydric alcohols such as mannitol, sorbitol and dulcitol. Inter alia, hexahydric alcohols are preferably used, and in particular, mannitol is suitably used.

The monohydric alcohols include, for example, methanol, ethanol and isopropyl alcohol, and among them, ethanol is preferably used.

The monosaccharides include, for example, pentose such as arabinose, xylose, ribose and 2-deoxyribose, and hexose such as glucose, fructose, galactose, mannose, sorbose, rhamnose and fucose, and among them, hexose is preferably used.

The oligosaccharides include, for example, trisaccharides such as maltotriose and raffinose saccharides, and tetrasaccharides such as stachyose, and among them, trisaccharides are preferably used.

The derivatives of monosaccharides, disaccharides and oligosaccharides include, for example, glucosamine, galactosamine, glucuronic acid, and galacturonic acid.

As the amino acid, any amino acid can be used as long as it is L-isomer and the examples include glycine,

leucine and arginine. Among them, L-arginine is preferably used.

These osmotic pressure regulating agents may be used alone or may be used by mixing them.

An osmotic pressure regulating agent is used at such a concentration that the osmotic pressure of the outer aqueous phase is about 1/50 to about 5 times, preferably about 1/25 to about 3 times, more preferably about 1/12 to about 2 times the osmotic pressure of a physiological saline.

Specifically, in the case where an osmotic pressure regulating agent is a nonionic substance, the concentration of the osmotic pressure regulating agent in the outer aqueous phase is about 0.01% to about 60% (w/w), preferably about 0.01 to about 40% (w/w), more preferably about 0.05 to about 30% (w/w), particularly preferably about 0.5 to about 1.5% (w/w). In the case where an osmotic pressure regulating agent is an ionic substance, a concentration obtained by dividing the above-described concentration by the whole ionic valency is used. The concentration of an osmotic regulating agent to be added is not necessary to be the solubility or lower, and a part of the agent may be in the dispersed state.

The dispersivity of the produced microcapsule can be improved by adding an osmotic pressure regulating agent to the outer aqueous phase. The extent of dispersivity is particularly not limited, but, for example, it is preferable that about 400 to 700 mg of the microcapsule can be dispersed in 1.5 mL of a dispersing medium for injection in less than two minutes.

Removal of an organic solvent may be performed in accordance with a known method. Such a method includes, for example, a method of removing the solvent under the normal pressure or gradually reduced pressure while stirred with a propeller-type stirrer, a magnetic stirrer or the like, and a method of removing the solvent using a rotary evaporator or the like while the vacuum degree and the temperature are regulated.

The sustained-release microcapsule thus obtained is collected by centrifugation, filtration, wet cyclone or the like, washed with distilled water repeatedly.several times to remove a free physiologically active substance, an emulsifier and the like which are adhered to the surface of the microcapsule. Subsequently, the washed microcapsule is dried under reduced pressure, or is redispersed in distilled water and then lyophilized to further remove an organic solvent.

During the producing process, in order to prevent particles from aggregating each other, an aggregation preventing agent may be added. The aggregation preventing agent includes, for example, water soluble polysaccharides such as mannitol, lactose, glucose and starches (for example, corn starch and the like), amino acids such as glycine, and proteins such as fibrin and collagen. Among them, mannitol is preferably used.

The amount of an aggregation preventing agent such as mannitol to be added is usually 0 to about 24% by weight of the total amount of the microcapsules.

The sustained-release microcapsule preferably contains an excipient. It is desired that the excipient is low in toxicity even when administered to a living body, is easily dried such as by lyophilization, and is rapidly dissolved when administered to a living body or is dissolved upon use. Such an excipient includes, for example, sugars, cellulose derivatives, amino acids, proteins, polyacrylic acid derivatives, organic salts, and inorganic salts. Two or more kinds of these excipients may be mixed and used at an appropriate ratio.

Herein, the sugars include, for example, D-mannitol, sodium alginate, fructose, dextran, dextrin, white sugar, D-sorbitol, lactose, glucose, maltose, starches and trehalose.

The cellulose derivatives include, for example, carboxymethylcellulose, hydroxypropylmethylcellulose, ethylcellulose, hydroxymethylcellulose, hydroxypropylcellulose, cellulose acetate phthalate, hydroxypropylmethylcellulose phthalate, and hydroxymethylcellulose acetate succinate.

The amino acids include, for example, glycine, alanine, tyrosine, arginine, and lysine.

The proteins include, for example, fibrin, collagen, and albumin.

The polyacrylic acid derivatives include, for example, sodium polyacrylate, and methacrylic acid/acrylic acid copolymers (Eudragit, Rohm, Co., Ltd., Germany).

The organic salts include, for example, sodium citrate, sodium tartrate, sodium carbonate, and potassium carbonate.

The inorganic salts include, for example, sodium chloride, potassium chloride, sodium phosphate, and potassium phosphate.

As the excipient, in addition to the above-mentioned excipients, water-soluble polymers in which the polymer that is the base of the sustained-release microcapsule is not dissolved, for example, polyvinylpyrrolidone and polyvinyl alcohol are also used.

The excipient is preferably a sugar, and inter alia, D-mannitol which is easily lyophilized and has little toxicity.

The amount of an excipient used is determined depending on the solubility of the excipient, the tonicity, viscosity, dispersivity and stability of a solution of the excipient and the like. In the case where the sustained-release microcapsule is dried, an excipient is used so that the content of the excipient in the dried sustained-release microcapsule is, for example, about 0.5 to about 99% (w/w), preferably about 1 to 90% (w/w), more preferably about 2 to about 60% (w/w). In the case where D-mannitol is used as an excipient, it is preferable that the content of the excipient in the dried sustained-release microcapsule is about 2 to about 40% (w/w), preferably about 15% (w/w).

By adding the excipient, the following excellent effects are obtained: 1) the frequency of contact and collision

of particles during and after drying the sustained-release microcapsule is reduced, and thereby uniformity of the particles at lyophilization is maintained, 2) the sustained-release microcapsule can be dried at the glass transition temperature or higher, and thereby water or an organic solvent can be removed more completely, and 3) the stability with time of the sustained-release microcapsule is improved, and thereby a sustained-release microcapsule which has better dispersivity, can be stored in any place including but not limited to cold places, and has a long term limit for use, for example, at room temperature is obtained.

In the present invention, the sustained-release microcapsule containing an excipient can be produced, for example, by mixing a microcapsule obtained by the above-described in-water drying method with an excipient. The microcapsule obtained by the above-described in-water drying method may be used after washed and then dried under reduced pressure, or after washed, redispersed in distilled water and then lyophilized. A method of mixing is not particularly limited, and for example, mixing is carried out by using a mixer.

The sustained-release microcapsule containing an excipient can be also produced by using an aqueous solution of an excipient as the outer aqueous phase when the W/O/W emulsion to be subjected to in-water drying is produced.

The sustained-release microcapsule containing an excipient is preferably produced by washing a microcapsule obtained by the in-water drying method, dispersing the washed microcapsule in distilled water in which an excipient has been dissolved or suspended, and then subjecting the dispersion to lyophilization or drying under reduced pressure. Alternatively, the washed microcapsule may be dispersed in distilled water, and an excipient may be dissolved or suspended in the obtained dispersion, followed by lyophilization or drying under reduced pressure. Inter alia, an uniform mixture is obtained by dispersing the washed microcapsule in distilled water in which an excipient had been dissolved, or by dispersing the washed microcapsule in distilled water, dissolving an excipient in the obtained dispersion and then lyophilizing the dispersion.

Furthermore, if desired, water and an organic solvent in the microcapsule can be removed more completely and, at the same time, sustained-release can be improved by heating the microcapsule obtained by the above-described in-water drying method to a temperature which is the glass transition temperature (Tg) or higher of the polymer used as a base and at which particles of the microcapsules are not attached to each other. At that time, it is preferable that an organic solvent is removed to less than about 1,000 ppm, preferably less than about 500 ppm, more preferably less than about 100 ppm of the organic solvent concentration in the microcapsule.

A glass transition temperature refers to an intermediate point glass transition temperature obtained when the temperature is raised at a heating rate of 10 or 20°C per minute using a differential scanning calorimeter (DSC).

The timing of heating is preferably after optional addition of an excipient and after lyophilization or drying under reduced pressure, but it is not particularly limited and for example, it may be after dispensing.

When the heating temperature is lower than the glass transition temperature of the polymer used as a base, removal of water or an organic solvent is insufficient in some cases. On the other hand, when the heating temperature is too high, the risk of fusion and deformation of the microcapsules, and degradation and deterioration of the physiologically active substance is increased. Thus, the heating temperature can not be unconditionally defined, but can be appropriately determined in view of physical properties (for example, molecular weight, stability and the like) of the polymer used as a base, the average particle diameters of the physiologically active substance and microcapsule, the heating time, the drying degree of the microcapsule, a method of heating and the like.

The heating temperature is preferably in the range from the glass transition temperature of the polymer used as a base to about 40°C higher temperature than the glass transition temperature, more preferably from the glass transition temperature of the polymer to 35°C higher temperature than the glass transition temperature, still more preferably from the glass transition temperature of the polymer to 25°C higher temperature than the glass transition temperature, particularly preferably from the glass transition temperature of the polymer to 20°C higher temperature than the glass transition temperature.

The heating time varies depending on the heating temperature, the amount of microcapsules to be treated and the like, and is generally about 6 to 120 hours, more preferably about 12 to 96 hours after the temperature of the microcapsule is reached to the predetermined temperature. The upper limit of the heating time is not particularly limited as long as the amounts of a remaining organic solvent and water become an acceptable amount or lower. However, under the glass transition temperature or higher, the microcapsule is softened, and deformed due to physical contact between the microcapsules or loading at lamination of the microcapsules. Therefore, it is preferable that the heating is rapidly terminated when the amounts of a remaining organic solvent and water become an acceptable amount or lower.

A method of heating is not particularly limited, and any method may be used as long as it can heat microcapsules uniformly. A preferable example of such a heating method includes a method comprising heating and drying under reduced pressure using a lyophilizing machine, a reduced pressure constant temperature machine or the like.

The sustained-release composition including a sustained-release microcapsule may be in the form of an injection, an implant, a oral preparation (for example, powder, granule, capsule, tablet, syrup, emulsion, suspension or the like), a transnasal preparation, a suppository (for example, rectal suppository, vaginal suppository or the like) or the like, and can be produced by a method usually known in the pharmaceutical field.

For example, an injection is produced by dispersing the above-described microcapsules into an aqueous or oily dispersion medium. An aqueous dispersion medium includes, for example, a solution in which an isotonic agent (for example, sodium chloride, glucose, D-mannitol, sorbitol, glycerin or the like), a dispersing agent (for example, Tween 80, HCO-50, HCO-60, carboxymethylcellulose, sodium alginate or the like), a preservative (for example, benzyl alcohol, benzalconium chloride, phenol or the like), a shoothing agent (for example, glucose, calcium gluconate, procaine hydrochloride or the like) or the like is dissolved. An oily dispersion medium includes, for example, olive oil, sesame oil, peanut oil, soybean oil, corn oil, and medium chain fatty acid glyceride.

The chamber of a prefilled syringe may be filled with the injection. Alternatuvely, the two different chambers of a so-called double chamber prefilled syringe (DPS) may be filled with a dispersion medium and the microcapsules separately.

Moreover, a more stable sustained-release injection is obtained by, in producing the injection, adding an excipient (for example, mannitol, sorbitol, lactose, glucose or the like) in addition to the above-described ingredients to the microcapsules, dispersing again it, lyophilizing or spraying-drying the dispersion to solidify it, and then adding distilled water for injection or a suitable dispersion medium to the solid before use.

An oral preparation can be produced, for example, by adding an excipient (for example, lactose, white sugar, starch or the like), a disintegrant (for example, starch, calcium carbonate or the like), a binder (for example, starch, gum arabic, carboxymethylcellulose, polyvinylpyrrolidone, hydroxypropylcellulose or the like), a lubricant (for example, talc, magnesium stereate, polyethylene glycol 6000 or the like) and the like to the above-described microcapsules, compressing and molding the mixture, and then, if necessary, coating it by means of a per se known method for the purpose of masking of taste, enteric coating or sustained-release. A coating agent includes, for example, hydroxypropylmethylcellulose, ethylcellulose, hydroxymethylcellulose, hydroxypropylcellulose, polyoxyehtylene glycol, Tween 80, Pluronic $F_{68}$, cellulose acetate phthalate, hydroxypropylmethycellulose phthalate, hydroxymethylcellulose acetate succinate, Eudragit (made by Rohm, Co., Ltd., Germany, methacrylic acid/acrylic acid copolymer) and pigments (for example, titanium dioxide, colcothar and the like).

A transnasal preparation may be in the form of a solid, a semi-solid, or liquid. A transnasal preparation in the form of a solid may be, for example, the above-described microcapsule as it is, but usually it can be produced by adding an excipient (for example, glucose, mannitol, starch, microcrystalline cellulose or the like), a thickening agent (for example, natural gums, cellulose derivatives, acrylic acid polymers or the like) and the like to the microcapsules and mixing them. For example, a transnasal preparation in the form of liquid can be produced in a similar manner to the case of the above-described injection. The transnasal preparation may contain a pH adjusting agent (for example, carbonic acid, phosphoric acid, citric acid, hydrochloric acid, sodium hydroxide or the like), an antiseptic agent (for example, parahydroxybenzoic acid esters, chlorobutanol, benzalconium chloride or the like) and the like.

A suppository may be oily or aqueous, and may be in the form of a solid, a semi-sold or liquid. A suppository is usually produced using an oily base, an aqueous base or an aqueous gel base. An oily base includes, for example, higher fatty acid glyceride [for example, cacao butter, Witepsol-series products (Dynamite Nobel), etc.], medium fatty acid [e.g., Miglyol-series products (Dynamite Nobel), etc.], vegetable oil (e.g., sesame oil, soybean oil, cottonseed oil, etc.) and the like. An aqueous base includes, for example, polyethylene glycol, propylene glycol and the like. An aqueous gel base includes, for example, natural gums, cellulose derivatives, vinyl polymers, acrylic acid polymers, and the like.

Specific examples of the sustained-release microcapsule include sustained-release microcapsules described in Example A1-A3.

The sustained-release composition, particularly the sustained-release microcapsule, is preferably an injection. In the case where the sustained-release composition is an injection, the particle diameter of the sustained-release composition as an injection may be in such a range that its dispersivity and needle penetrating property are satisfied, and it is, for example, an average diameter of about 0.1 to about 1,000 $\mu$m, preferably about 1 to about 300 $\mu$m, more preferably about 5 to about 150 $\mu$m.

In producing the sustained-release composition, the desolvation rate is high. For example, the concentration of remaining methylene chloride in the composition after in-water drying (for example, after three hours) is usually about 2,000 ppm to about 20,000 ppm. Therefore, the sustained-release composition is excellent in desolvation property.

Furthermore, the sustained-release composition has an excellent characteristic of a slow sedimentation rate. The sedimentation rate can be determined, for example, by putting 50 mg of the sustained-release microcapsule powder of the present invention in a vial, suspending the powder in 5 mL of a dispersion medium, dispersing about 40 $\mu$L of the obtained suspension in 5 mL of a dispersion medium, and measuring NTU with a turbi-dimeter. The sustained-release composition of the present invention has a characteristic that it takes a long time for the turbidity to reach 50% of that immediately after suspending when the composition is suspended.

The sustained-release composition is less toxic and thus can be safely administered to mammals (mouse, rat, dog, cat, sheep, pig, horse, bovine, monkey, human and the like).

The dosage of the sustained-release composition varies depending on the kind and content of a physiologically active substance, duration of sustained-release of a physiologically active substance, the targeted animal, the purpose

of administration, and the like, but may be an effective amount of the physiologically active substance.

In the case where the sustained-release composition is used to human, the dosage can be appropriately selected from the range from about 1 mg to about 10 g, preferably from about 10 mg to about 2 g per time for an adult (body weight 50kg). In the case where the sustained-release composition is an injection, the volume of the suspension to be administered can be appropriately selected from the range from about 0.1 to about 5 mL, preferably from about 0.5 to about 3 mL.

Particularly, when the physiologically active substance is a LH-RH derivative such as leuprorelin or leuprorelin acetate, the sustained-release composition is effective as an agent for preventing or treating hormone-dependent disease (for example, prostate cancer, prostatomegaly, endometriosis, hysteromyoma, metrofibro-ma, precocious puberty, dysmenorrhea or breast cancer, or the like), a contraceptive agent, an agent for preventing the postoperative recurrence of premenopausal breast cancer, or the like.

The monthly dosage of the sustained-release composition for an adult (body weight 50 kg) is, for example, about 1.88 to about 15 mg of the physiologically active substance. In the case where the sustained-release composition is administered to domestic animals (for example, dog, cat, sheep, pig, horse, bovine, monkey and the like) for the purpose of contraception or softening of meat, the dosage is determined by the measured clearance of animals to be administered. For example, in the case where an animal to be administered is a dog, the monthly dosage of the sustained-release composition is, for example, about 0.03 to about 3.0 mg/kg of the physiologically active substance.

Effect of Invention

Since the desolvation rate is high in producing the sustained-release microcapsule, the sedimentation rate is slow when the sustained-release microcapsule is suspended, and in addition, the sustained-release microcapsule has excellent spherical property and/or needle penetrating property, the sustained-release microcapsule can exert the excellent sustained-release property. Furthermore the sustained-release microcapsule is effective as an agent for preventing or treating prostate cancer, prostatomegaly, endometriosis, hysteromyoma, metrofibroma, precocious puberty, dysmenorrhea or breast cancer, a contraceptive agent, or an agent for preventing the postoperative recurrence of premenopausal breast cancer.

Hereinafter, the present invention will be further specifically illustrated by reference to Examples, which do not limit the scope of the present invention.

The % (percentage) represents % by weight unless it is especially noted.

In Examples, a lactic acid-glycolic acid copolymer comprising 75 mol% of DL-lactic acid and 25 mol% of glycolic acid was used.

Examples

Reference Example 1

Measurement of weight-average molecular weight (Mw) of polymer

About 0.05 g of a polymer is weighed, tetrahydrofuran (THF) is added to dissolve it to 5 mL, and a sample solution is obtained.

Each about 0.1 g of polystyrene standard products (F-10, F-2, A-5000 and A-1000) having known molecular weights is weighed, THF is added to dissolve it to 40 mL, and a standard solution A is obtained. Each about 0.1 g of polystyrene standard products (F-4, F-1, A-2500 and A-500) having known molecular weights is weighed, THF is added to dissolve it to 40 mL, and a standard solution B is obtained.

100 $\mu$L of the sample solution and standard solutions A and B are tested by a gel permeation chromatography method under the following conditions. A molecular weight calibration curve is prepared from the molecular weights of respective polystyrene standard products and their retention times. Next, the peak height (Hi) of eluted component obtained from the sample solution is measured, and its molecular weight (Mi) is obtained from its retention time and the molecular weight calibration curve. The weight-average molecular weight (Mw) of the polymer is determined by the following equation.

[Calculation Equation] $$Mw = \Sigma(Hi \times Mi)/\Sigma Hi$$

[Test Condition]

Detector: differential refractometer (having performance equivalent to that of HLC-8120 GPC system)

Column: TSK guardcolumn $H_{HR}$-L (40×6.0 mm i.d.)
TSKgel G4000$H_{HR}$ (300×7.8 mm i.d.)
TSKgel G3000$H_{HR}$ (300×7.8 mm i.d.)
TSKgel G2000$H_{HR}$ (300×7.8 mm i.d.)
TSKgel G1000$H_{HR}$ (300×7.8 mm i.d.)

These columns are connected in a series in the order of reduction in pore diameter of fillers (or those having the similar performance can be used).

Column temperature: constant temperature around 50°C Mobile phase: THF
Flow rate: 1.0 mL/min

[System suitability]

(1) Performance of System:

When the system is operated on 100 μL of the standard solution A under the above-described conditions, the separation degree between the peak of F-10 and the peak of F-2 is 2.0 or more, and the theoretical step number and symmetry coefficient of both peaks are 8000 steps or more and 1.5 or less, respectively.

(2) Reproducibility of Test:

When the test is repeated twice on 100 μL of the standard solution A under the above-described conditions, the relative standard deviation between retention times of respective peaks is 3.3% or less.

[Operation method]

Standard solution: The solution is stable at room temperature (about 25°C) for at least 24 hours after preparation. In addition, the solution is stable for at least 7 months in a refrigerator (about -18°C) after preparation.
Sample solution: The solution is stable at room temperature (about 25°C) for at least 24 hours after preparation.
Range of area measurement: 48 minutes (injection interval is 50 minutes).
Molecular weight calibration curve: it is prepared by polygonal line.

In addition to weight-average molecular weight (Mw), number-average molecular weight [$Mn = \Sigma Hi/\Sigma(Hi/Mi)$] is also measured.

[Injection Order].

(1) A test is repeated twice on the standard solution A, and it is confirmed that the first test adapts to the rule of performance of the system. Retention times of respective peaks are found, and it is confirmed that these adapt to the rule of reproducibility of the test (the relative standard deviation between retention times of respective peaks is 3.3% or less).
(2) The standard solution B is injected, and retention times of respective peaks are found.
(3) A mobile phase is injected, a carryover of all peaks of the standard solution B injected in (2) is checked, and it is confirmed that peak area value adapts to the rule (10% or less).
(4) Measurement of the sample solution (maximum 12).
(5) A mobile phase is injected, a carryover of the sample solution finally injected in (4) is checked, and it is confirmed that that peak area value adapts to the rule (10% or less).
(6) The standard solutions A and B are injected, and retention times of respective molecular weights are found.

(7) A molecular weight calibration curve is prepared from retention times of the standard solution A finally injected in (1), the standard solution B injected in (2) and the standard solutions A and B injected in (6), and the weight average molecular weight (Mw) of the sample is calculated, provided that it is confirmed that the relative deviation of retention times [RD: % of a difference (absolute value) from an average value of any retention time relative to an average value] of the standard solution A finally injected in (1) and the standard solution A injected in (6) is 3.3% or less. When it is not adapted, all data between system checks is invalidated, and the test is performed again from the step (1) (provided that it is not necessary to investigate the performance of the system).

[Reagent/Reagent solution]

(I) GPC method (1)

Polystyrene standard products: TSK standard polystrene/ manufactured by Tosoh Corporation.

As Mw of the polystyrene standard products, values evaluated by a GPC method are used.

| Type | Mw |
|---|---|
| F-10 | 98900 |
| F-4 | 37200 |
| F-2 | 17100 |
| F-1 | 9490 |
| A-5000 | 5870 |
| A-2500 | 2500 |
| A-1000 | 1051 |
| A-500 | 495 |

Tetrahydrofuran: for liquid chromatography, manufactured by Wako Pure Chemical Industries, Ltd.

(II) GPC method (2)

Polystyrene standard products: TSK standard polystrene/ manufactured by Tosoh, co., Ltd.

As Mw of the polystyrene standard products, in the case of A-1000 and A-500, values evaluated by a GPC method are used, and in the other cases, values evaluated by a light scattering method are used.

| Type | Mw |
|---|---|
| F-10 | 96400 |
| F-4 | 37900 |
| F-2 | 18100 |
| F-1 | 10200 |
| A-5000 | 5970 |
| A-2500 | 2630 |
| A-1000 | 1051 |
| A-500 | 495 |

Tetrahydrofuran: for liquid chromatography, manufactured by Wako Pure Chemical Industries, Ltd.

Example A1

To 15 g of leuprorelin acetate (content 98.5%), 13 g of distilled water for injection was added and leuprorelin acetate was dissolved in a warm bath at 80°C to prepare an A solution.

To 327 g of a lactic acid-glycolic acid copolymer (content 98.6%) (lactic acid/glycolic acid = 75/25, Mw = 10300, Mn = 4000, Mw/Mn ratio = 2.6 (value by the GPC method (1) of Reference Example 1), 540 g of methylene chloride was added and the copolymer was dissolved to prepare a B solution.

To 50 g of polyvinyl alcohol, 5 L of distilled water for injection was added and polyvinyl alcohol was dissolved to prepare a C solution.

To the A solution, 328 g of the B solution that had been adjusted to 30°C was added, and the mixture was rough emulsified by vibrating a container. After rough emulsification, the temperature of the resulting rough emulsion was 32°C. The rough emulsion was emulsified at 10,000 rpm for 1 minute twice with a propeller-type stirrer (Autohomomixer/M-type, Tokushukika Kogyo Co., Ltd.) to prepare a W/O emulsion. The W/O emulsion was cooled to 19°C, and the viscosity was adjusted. From the W/O emulsion, about 7 g was taken as a sample for measurement of viscosity with a vibration viscometer (VM-100, Yamaichi Electronics Co., Ltd.). The viscosity of the sample was 1430 cp. 2.5 L of the C solution was diluted with distilled water for injection to 25 L and then adjusted to 18°C. Thereto the above-described W/O emulsion was added. The mixture was emulsified at 7,000 rpm with a propeller-type stirrer (Homomic line flow/100-type, Tokushukika Kogyo Co., Ltd.) to prepare a W/O/W emulsion. After the above-described W/O/W emulsion was stirred for 3 hours, methylene chloride was removed by in-water drying method. After microcapsules with large particle diameter were filtered off through a 75 μm sieve, microcapsules were collected at 2,000 rpm by a successional centrifuge separator (H-600S / made by Kokusan). The microcapsules were dispersed again in distilled water for injection. To the dispersion, 18 g (18.0 g) of mannitol as an aggregation prevention agent was added and dissolved. The dispersion thus obtained was lyophilized to prepare microcapsule powder containing leuprorelin acetate.

Mannitol was used in such an amount that the microcapsule powder contained about 15% of mannitol. The obtained microcapsule powder contained 8.3% of leuprorelin acetate, and was in a spherical shape. It was found that the obtained microcapsule powder continuously released leuprorelin acetate over one month by an animal test (rat).

Example A2

To 16 g of leuprorelin acetate (content 98.5%), 16 g of distilled water for injection was added and leuprorelin acetate was dissolved in a warm bath at 80°C to prepare an A solution.

To 334 g of a lactic acid-glycolic acid copolymer (content 98.6%) (lactic acid/glycolic acid = 75/25, Mw = 10300, Mn = 4000, Mw/Mn ratio = 2.6 (value by the GPC method (1) of Reference Example 1), 540 g of methylene chloride was added and the copolymer was dissolved to prepare a B solution.

To 50 g of polyvinyl alcohol, 5 L of distilled water for injection was added and polyvinyl alcohol was dissolved to prepare a C solution.

To the A solution, 330 g of the B solution that had been adjusted to 30°C was added, and the mixture was rough emulsified by vibrating a container. After rough emulsification, the temperature of the resulting rough emulsion was 32°C. The rough emulsion was emulsified at 10,000 rpm for 1 minute twice with a propeller-type stirrer (Autohomomixer/M-type, Tokushukika Kogyo Co., Ltd.) to prepare a W/O emulsion. The liquid temperature of a W/O emulsion after the emulsification was 30.7°C. The W/O emulsion was cooled to 18°C, and the viscosity was adjusted. From the W/O emulsion, about 7 g was taken as a sample for measurement of viscosity with a vibration viscometer (VM-100, Yamaichi Electronics Co., Ltd.). The viscosity of the sample was 1880 cp. 2.5 L of the C solution was diluted with distilled water for injection to 25 L and then adjusted to 18°C. Thereto the above-described W/O emulsion was added. The mixture was emulsified at 7,000 rpm with a propeller-type stirrer (Homomic line flow/100-type, Tokushukika Kogyo Co., Ltd.) to prepare a W/O/W emulsion. After the above-described W/O/W emulsion was stirred for 3 hours, methylene chloride was removed by in-water drying method. After microcapsules with large particle diameter were filtered off through a 75 μm sieve, microcapsules were collected at 2,000 rpm by a successional centrifuge separator (H-600S / made by Kokusan). The microcapsules were dispersed again in distilled water for injection. To the dispersion, 18g of mannitol as an aggregation prevention agent was added and dissolved. The dispersion thus obtained was lyophilized to prepare microcapsule powder containing leuprorelin acetate.

Mannitol was used in such an amount that the microcapsule powder contained about 15% of mannitol. The obtained microcapsule powder contained 8.2% of leuprorelin acetate, and was in a spherical shape. It was found that the obtained microcapsule powder continuously released leuprorelin acetate over one month by an animal test (rat).

Example A3

To 15 g of leuprorelin acetate (content 98.5%), 13 g of distilled water for injection was added and leuprorelin acetate was dissolved in a warm bath at 80°C to prepare an A solution.

To 334 g of a lactic acid-glycolic acid copolymer (content 98.6%) (lactic acid/glycolic acid = 75/25, Mw = 10300, Mn = 4000, Mw/Mn ratio = 2.6 (value by the GPC method (1) of Reference Example 1), 540 g of methylene chloride was added and the copolymer was dissolved to prepare a B solution.

To 50 g of polyvinyl alcohol, 5 L of distilled water for injection was added and polyvinyl alcohol was dissolved to prepare a C solution.

To the A solution, 330 g of the B solution that had been adjusted to 30°C was added, and the mixture was rough emulsified by vibrating a container. After rough emulsification, the temperature of the resulting rough emulsion was 32°C. The rough emulsion was emulsified at 10,000 rpm for 1 minute twice with a propeller-type stirrer (Autohomomixer/M-type, Tokushukika Kogyo Co., Ltd.) to prepare a W/O emulsion. The liquid temperature of a W/O emulsion after the emulsification was 30.7°C. The W/O emulsion was cooled to 18°C, and the viscosity was adjusted. From the W/O emulsion, about 7 g was taken as a sample for measurement of viscosity with a vibration viscometer (VM-100, Yamaichi Electronics Co., Ltd.). The viscosity of the sample was 1880 cp. 2.5 L of the C solution was diluted with distilled water for injection to 25 L and then adjusted to 18°C. Thereto the above-described W/O emulsion was added. The mixture was emulsified at 7,000 rpm with a propeller-type stirrer (Homomic line flow/100-type, Tokushukika Kogyo Co., Ltd.) to prepare a W/O/W emulsion. After the above-described W/O/W emulsion was stirred for 3 hours, methylene chloride was removed by in-water drying method. After microcapsules with large particle diameter were filtered off through a 75 μm sieve, microcapsules were collected at 2,000 rpm by a successional centrifuge separator (H-600S / made by Kokusan). The microcapsules were dispersed again in distilled water for injection. To the dispersion, 18g of mannitol as an aggregation prevention agent was added and dissolved. The dispersion thus obtained was lyophilized to prepare microcapsule powder containing leuprorelin acetate.

Mannitol was used in such an amount that the microcapsule powder contained about 15% of mannitol. The obtained microcapsule powder contained 8.2% of leuprorelin acetate, and was in a spherical shape. It was found that the obtained microcapsule powder continuously released leuprorelin acetate over one month by an animal test (rat).

Example A4

In an eggplant-type Kolben, 119.1 g of leuprorelin acetate was weighted. Thereto 120 g of water for injection was added and leuprorelin acetate was completely dissolved. Thereto 975g of a lactic acid-glycolic acid copolymer (lactic acid/glycolic acid ratio = 75:25, Mw = about 10,400, Mn = about 4,100, Mw/Mn = 2.5 (value by the GPC method (1) of Reference Example 1)) dissolved in 1600 g of dichloromethane was added. The mixture was emulsified by stirring at 5800rpm for 10 minutes with an autominimixer to prepare a W/O emulsion. After this W/O emulsion was cooled to about 19°C, it was poured into 200 L of a 0.1% (w/w) polyvinyl alcohol aqueous solution (EG-40, made by Nippon Synthetic Chemical Industry Co., Ltd.) which had been previously adjusted to about 19°C. The mixture was emulsified by stirring at about 7000 rpm using HOMOMIC LINE FLOW (manufactured by Tokushukika) to prepare a W/O/W emulsion. This W/O/W emulsion was stirred at about 2500 rpm for 3 hours to volatilize or diffuse dichloromethane into the outer aqueous phase and to solidify the oil phase. After passed through a sieve having an opening of 75 μm, microcapsules were continuously settled with a centrifuge separator at about 2000 rpm and then collected. The collected microcapsules were dispersed into a small amount of distilled water and then passed through a sieve having an opening of 90 μm. Thereto 174.5 g of mannitol was added and dissolved. This was lyophilized to prepare microcapsule powder containing leuprorelin acetate. The microcapsule powder contained 8.5% of leuprorelin acetate.

Industrial Applicability

Since the desolvation rate is high in producing the sustained-release microcapsule, the sedimentation rate is slow when the sustained-release microcapsule is suspended, and in addition, the sustained-release microcapsule has excellent spherical property and/or needle penetrating property, the sustained-release microcapsule can exert the excellent sustained-release property. Furthermore, the sustained-release microcapsule is effective as an agent for preventing or treating prostate cancer, prostatomegaly, endometriosis, hysteromyoma, metrofibroma, precocious puberty, dysmenorrhea or breast cancer, a contraceptive agent, or an agent for preventing the postoperative recurrence of premenopausal breast cancer.

## Claims

**1.** A process for producing a sustained-release microcapsule which comprises (i) a physiologically active substance and (ii) a lactic acid-glycolic acid copolymer in which the weight-average molecular weight (Mw) is about 8,000 to about 11,500, the ratio of the weight-average molecular weight (Mw) to the number-average molecular weight (Mn) is 2.3 to 3.1, and the compositional molar ratio of lactic acid to glycolic acid is 99.9/0.1 to 60/40, or a salt thereof, and which does not contain a drug retaining substance, and wherein the physiologically active substance is an LH-RH derivative; which comprises mixing (i) a solution containing a physiologically active substance and not containing a drug retaining substance and (ii) a solution adjusted to about 25 to about 35°C and containing a lactic acid-glycolic

acid copolymer in which the weight-average molecular weight (Mw) is about 8,000 to about 11,500, the ratio of the weight-average molecular weight (Mw) to the number-average molecular weight (Mn) is 2.3 to 3.1, and the compositional molar ratio of lactic acid to glycolic acid is 99.9/0.1 to 60/40, or a salt thereof to prepare a W/O emulsion of about 25 to about 35°C; cooling the W/O emulsion to about 15 to about 20°C; dispersing the W/O emulsion into an aqueous phase to prepare a W/O/W emulsion; and then subjecting the W/O/W emulsion to in-water drying.

## Patentansprüche

1. Verfahren zur Herstellung einer Mikrokapsel mit nachhaltiger Wirkstofffreisetzung, die (i) eine physiologisch aktive Substanz und (ii) ein Milchsäure-Glycolsäure-Copolymer, bei dem das Gewichtsmittel des Molekulargewichts (Mw) etwa 8000 bis etwa 11 500 beträgt, das Verhältnis von Gewichtsmittel des Molekulargewichts (Mw) zu Zahlenmittel des Molekulargewichts (Mn) 2,3 bis 3,1 beträgt und das kompositionale Stoffmengenverhältnis von Milchsäure zu Glycolsäure 99,9/0,1 bis 60/40 beträgt, oder ein Salz davon umfasst und keine wirkstoffzurückhaltende Substanz enthält, wobei die physiologisch aktive Substanz ein LH-RH-Derivat ist, umfassend das Mischen von (i) einer Lösung, die eine physiologisch aktive Substanz enthält und keine wirkstoffzurückhaltende Substanz enthält, und (ii) einer Lösung, die auf etwa 25 bis etwa 35 °C eingestellt ist und ein Milchsäure-Glycolsäure-Copolymer, bei dem das Gewichtsmittel des Molekulargewichts (Mw) etwa 8000 bis etwa 11 500 beträgt, das Verhältnis von Gewichtsmittel des Molekulargewichts (Mw) zu Zahlenmittel des Molekulargewichts (Mn) 2,3 bis 3,1 beträgt und das kompositionale Stoffmengenverhältnis von Milchsäure zu Glycolsäure 99,9/0,1 bis 60/40 beträgt, oder ein Salz davon enthält, zur Herstellung einer W/O-Emulsion von etwa 25 bis etwa 35 °C; Abkühlen der W/O-Emulsion auf etwa 15 bis etwa 20 °C; Dispergieren der W/O-Emulsion in einer wässrigen Phase zur Herstellung einer W/O/W-Emulsion; und dann Trocknen der W/O/W-Emulsion durch In-Wasser-Trocknung.

## Revendications

1. Procédé de production d'une microcapsule à libération prolongée, qui comprend (i) une substance physiologiquement active et (ii) un copolymère d'acide lactique-acide glycolique ayant une masse moléculaire moyenne en masse (Mw) d'environ 8 000 à environ 11 500, un rapport de la masse moléculaire moyenne en masse (Mw) à la masse moléculaire moyenne en nombre (Mn) de 2,3 à 3,1, et un rapport molaire compositionnel de l'acide lactique à l'acide glycolique de 99,9/0,1 à 60/40, ou un sel de celui-ci, et qui ne comprend pas de substance de rétention du médicament, et dans laquelle la substance physiologiquement active est un dérivé de LH-RH ; qui comprend le mélange (i) d'une solution contenant une substance physiologiquement active et ne comprenant pas de substance de rétention du médicament et (ii) d'une solution ajustée à environ 25 à environ 35°C et contenant un copolymère d'acide lactique-acide glycolique ayant une masse moléculaire moyenne en masse (Mw) d'environ 8 000 à environ 11 500, un rapport de la masse moléculaire moyenne en masse (Mw) à la masse moléculaire moyenne en nombre (Mn) de 2,3 à 3,1, et un rapport molaire compositionnel de l'acide lactique à l'acide glycolique de 99,9/0,1 à 60/40, ou un sel de celui-ci, pour préparer une émulsion eau dans l'huile d'environ 25 à environ 35°C ; le refroidissement de l'émulsion eau dans l'huile à environ 15 à environ 20°C ; la dispersion de l'émulsion eau dans l'huile dans une phase aqueuse pour préparer une émulsion eau dans l'huile dans l'eau ; et ensuite la soumission de l'émulsion eau dans l'huile dans l'eau à un séchage dans l'eau.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 60100516 A
- JP 62201816 A
- JP 62054760 A
- JP 61028521 A
- JP 6192068 A
- JP 4218528 A
- WO 03002091 A
- US 3853837 A
- US 4008209 A
- US 3972859 A
- GB 1423083 A
- GB 2213737 A
- US 4024248 A
- DE 2438352
- JP 51041359 A
- US 4010125 A
- JP 52031073 A
- US 4100274 A
- JP 52136172 A
- US 4234571 A
- JP 55164663 A
- JP 63264498 A
- JP 6425794 A
- US 4569967 A
- US 4248439 A
- WO 9118016 A
- BE 897455
- JP 59059654 A
- US 4086219 A
- US 4124577 A
- US 4253997 A
- US 4317815 A
- JP 3101695 A
- EP 413209 A
- WO 9415587 A


### Non-patent literature cited in the description


- *AAPS Pharmsci,* 1999, vol. 1 (3
- *European Journal of Pharmaceutics and Biopharmaceutics,* 2000, vol. 50, 263-270
- *Proceedings of the National Academy of Sciences of the United States of America,* 1981, vol. 78, 6509-6612
- *Journal of Medicinal Chemistry,* 1992, vol. 35, 3942
- Encyclopedic Handbook of Biomaterials and Bioengineering Part A: Materials. Marcel Dekker, Inc, 1995, vol. 2
- *J. Controlled Release,* 1996, vol. 41, 249-257